# EUROPEAN PATENT APPLICATION

(11) **EP 2 500 425 A1**
(43) Date of publication of application: **19.09.2012**
(21) Application number: 10830064.1
(22) Date of filing: 12.11.2010
(51) Int. Cl.: C12N 11/02, C12N 5/0775

(54) **PARTICLE-CONTAINING CELL AGGREGATE**

(30) Priority: 13.11.2009 JP 2009260192; 08.03.2010 JP 2010050399
(71) Applicant: Hitachi High-Technologies Corporation, Tokyo 105-8717 (JP)
(72) Inventor: TABATA, Yasuhiko, Kyoto-shi Kyoto 612-8043 (JP)
(74) Representative: Strehl Schübel-Hopf & Partner
(86) International application number: PCT/JP2010/070630
(87) International publication number: WO 2011/059112

(57) **Abstract**

The present invention relates to a microsphere-containing cell aggregate including: hydrogel microspheres being obtained by chemical cross-linking of one or more water-soluble synthetic macromolecules selected from the group consisting of water-soluble synthetic polymers, polysaccharides, and proteins; and cells. The present invention also relates to a method for producing the microsphere-containing cell aggregate.

## Description

### Technical Field

The present invention relates to cell aggregates containing microspheres which promote cell viability and biological functions, and to methods for culturing the cell aggregate.

### Background Art

Progress in stem cell biomedical research has led to accumulation of fundamental findings on cell differentiation and expression of biological functions. In addition, adult (tissue) stem cells are collected from various biological tissues. The adult stem cells, together with embryonic stem (ES) cells and induced pluripotent stem (iPS) cells, have increasingly become applicable to research, drug discovery, and treatment which use these cells. This trend not only facilitates biomedical findings on stem cell proliferation, differentiation, and biological functions, but also is largely involved with achieving tissue formation from cells by artificially regulating cell differentiation and biological functions, and with improving drug discovery research using human cells and therapeutic efficacy using cell transplantation.

In general, cells rarely present in a single cell state *in vivo,* but live by means of a cell-cell interaction or cell-extracellular matrix interaction, which exerts their biological functions. In view of the above situation, realization of cell self-assembly and artificial tissue formation will advance biomedical research on cellular functions as well as will increase therapeutic efficacy using cell transplantation. The minimal unit of a body is a cell. However, in view of biological tissues and organs, the biological function unit is a cell aggregate (Hisao FUJITA and Tsuneo FUJITA, "HYOUJYUN SOSIKIGAKU (Standard Histology)", itemized discussion, published by Igaku-Shoin Ltd., 1992). Until now, there has been a publication (Landry J et al., J Cell Biol, 1985) reporting that formation of a cell aggregate (spheroid) created by self-assembly of hepatocytes significantly enhances a liver function compared with single hepatocytes without having a cell-cell interaction. It has been found that: compared with single cell culture, aggregated cell culture significantly increases cell-derived fibronectin production (Glimelius B et al., APMIS, 1988); and formation of an embryoid body (EB) promotes ES cell differentiation (Smith AG et al., Nature, 1988). When such a background is taken into consideration, the cell self-assembly is a critical technology in aspects of cell biology and cell transplantation.

Up to now, the cell self-assembly has been known to be facilitated by designs of a culture substrate (Mori R et al., J Biosci Bioeng, 106(3), 237-242, 2008) and a culture method (Kurosawa H, J Biosci Bioeng, 104(4), 294-299, 2007). As expected, cell proliferation, differentiation, and/or expression of biological functions are increased by the aggregation. However, the more increasingly the cells proliferate, the larger the size of the cell aggregate becomes. As a result, nutrition and oxygen supply into the inside of the cell aggregate and waste product removal from the inside of the aggregate are insufficient. Hence, this has led to a problem that continuous culture is impossible. Because of this, it is not allowed to carry out long-term culture which is indispensable for examinations in differentiation, tissue formation, and expression of cellular functions which are based on a cell-cell interaction. This has been an obstacle for progress in biomedical research on stem cells. In addition, cells whose gene is manipulated are cultured on microcarrier particles. Accordingly, microcarrier culture has been progressing which promotes production of a useful product such as a bioactive protein. In the microcarrier culture method, however, cells can proliferate only on the microcarrier surface, which causes a limitation of the number of cells that can proliferate thereon. Unfortunately, this causes a lower efficiency of producing a useful substance. Recently, as one of methods to solve the above problems, a culture method which employs a cell aggregate has been examined. Compared with the microcarrier method, this method can increase the number of cells, and can also increase productivity of cells producing an important useful substance (Nam J. H., et al., Biotech Prog., 23(3), p.652-660, 2007). Unfortunately, even in this case, as the size of the cell aggregate increases, cells inside the aggregate become weaker. This causes a decreased productivity of the cells producing the useful substance.

An *in vitro* cell assembly technique and a subsequent technique which facilitates organization of cells by applying the forgoing technique to two different cells indicate a future direction that current embryology and cell biology are heading to. The cell assembly is one of the key techniques. In view of the above background, desired is development of designs and methodologies to keep continuing culture even when the size of the cell aggregate increases.

### Citation List

### Non Patent Literature

Non Patent Literature 1: Mori R et al., J Biosci Bioeng, 106(3), p.237-242, 2008.
Non Patent Literature 2: Kurosawa H, J Biosci Bioeng, 104(4), p.294-299, 2007.
Non Patent Literature 3: Nam J. H. et al., Biotech Prog., 23(3), p.652-660, 2007.

### Summary of Invention

It is an object of the present invention to provide a method for preparing a cell aggregate capable of continuing long-term stable culture, and to provide a material therefor.

The present inventor has conducted intensive research to solve the above problems and has found that culture of cells with bioabsorbable particles produces a microsphere-containing cell aggregate. A microsphere-containing cell aggregate as prepared in accordance with the present invention can make it easy for substances to diffuse and exchange between the inside and the outside of the cell aggregate. This can achieve long-term culture of the aggregated cells, and can improve cellular functions and can promote differentiation. This technique advances biomedical research on cells using a cell aggregate and drug discovery research investigating drug metabolism and toxicity by using cells. In addition, this technique should enhance productivity of a cell aggregate producing a useful substance. A cell aggregate technique using this particle can apply to a hybrid artificial organ in which cells having a function of liver, pancreatic, or kidney cells are encapsulated with a hydrogel so as to achieve immunoprotection. In this case, the encapsulation causes a limitation of supplying nutrients and enzymes to the cells residing inside by diffusion, and also causes poor removal of waste products by diffusion. These phenomena exacerbate a cellular environment. In order to solve the above problems, it is effective to prepare a cell aggregate including these cells and microspheres to enhance their cellular functions. This allows for an increase in therapeutic efficacy.

Specifically, the present invention provides a microsphere-containing cell aggregate comprising cultured cells and gelatin hydrogel microspheres. In a preferable aspect of the present invention, the gelatin hydrogel microsphere comprises a cell growth factor. The present invention also provides a method for producing a microsphere-containing cell aggregate, the method comprising the step of culturing cells in a culture medium comprising gelatin hydrogel microspheres.

In the microsphere-containing cell aggregate, the microsphere presence increases efficiencies of supplying nutrients and oxygen into the inside of the cell aggregate and of removing waste products from the inside of the cell aggregate, which induces better cell conditions. As a result, a cell-cell interaction may be efficiently achieved in a manner similar to that of *in vivo* conditions. Thus, long-term cell culture seems to be able to be accomplished. The long-term culture further enhances cell differentiation and functional expression. This should increasingly develop such research.

The present application claims priority of JP Patent Application No. 2009-260192 and No. 2010-050399, whose specification and/or drawings are herein incorporated by reference.

### Brief Description of Drawings

[Figure 1] Figure 1 is a graph showing viable cell counts when cells are cultured alone or with gelatin hydrogel microspheres.
[Figure 2] Figure 2 is a graph showing viable cell counts in microsphere-containing aggregates when the cell aggregates are formed by changing a ratio of the number of cells to the number of microspheres.
[Figure 3] Figure 3 is pictures showing BrdU incorporation into a microsphere-containing cell aggregate. BrdU immunostaining (left); hematoxylin counterstaining of the same section (right).
[Figure 4] Figure 4 is a graph showing production of sulfated glycosaminoglycan (sGAG) by a microsphere-containing cell aggregate and a microsphere-free cell aggregate. Black bar:microsphere-containing cell aggregate; white bar: microsphere-free cell aggregate.
[Figure 5] Figure 5 is nuclear staining images of the cross sections of a microsphere-containing cell aggregate.
[Figure 6]Figure 6 is a graph showing viable cell counts of cell aggregates when a proportion of the number of plated cells/the number of added microspheres is modified at their culture. A star mark, p < 0.05; there is a significant difference with a microsphere-free agglomerate. #, p < 0.05; there is a significant difference with an agglomerate having the number of added microspheres of 1 x 10². *, p < 0.05; there is a significant difference with an agglomerate having the number of added microspheres of 1 x 10³.
[Figure 7] Figure 7 is a graph showing viable cell counts of cell aggregates when the size of microsphere varies at their culture. A star mark, p < 0.05; there is a significant difference with a microsphere-free agglomerate. †, p < 0.05; there is a significant difference with an agglomerate having a size of swelled microsphere of 17.67 ± 7.4 µm. ‡, p < 0.05; there is a significant difference with an agglomerate having a size of swelled microsphere of 47.9 ± 22.2 µm.
[Figure 8] Figure 8 is a graph showing glucose consumption in microsphere-containing cell aggregates. A star mark, p < 0.05; there is a significant difference with a microsphere-free agglomerate. †, p < 0.05; there is a significant difference with an agglomerate having a size of swelled microsphere of 17.67 ± 7.4 µm. ‡, p < 0.05; there is a significant difference with an agglomerate having a size of swelled microsphere of 47.9 ± 22.2 µm.
[Figure 9] Figure 9 is a graph showing a ratio of L-lactic acid yield/glucose consumption in microsphere-containing cell aggregates. A star mark, p < 0.05; there is a significant difference with a microsphere-free agglomerate.

### Description of Embodiments

Gelatin hydrogel microspheres as used in the present invention are a microparticulate gelatin hydrogel obtained by chemical cross-linking between gelatin molecules by using various chemical cross-linkers. Gelatin can be obtained by having collagen, collected from an animal or plant, denatured using various treatments such as alkaline hydrolysis, acid hydrolysis, and enzymatic hydrolysis. The gelatin may employ a denatured product of recombinant collagen.

Regardless of the degree of cross-linking and their size of the hydrogel microspheres, the hydrogel contains a large amount of water. Consequently, water-soluble nutritions, enzymes, and waste products easily diffuse. As long as having the above characteristics of the hydrogel, microspheres which can be used in the present invention can employ microspheres made of any material. The microspheres include a hydrogel in which water-soluble synthetic polymers, including polyacrylamide, polyacrylic acid, polyhydroxyethyl methacrylate, and polyvinyl alcohol, polysaccharides, and/or proteins are subjected to chemical cross-linking. Examples of the polysaccharides include, but are not limited to, glycosaminoglycan such as hyaluronic acid and chondroitin sulfate, starch, glycogen, agarose, pectin, cellulose, and the like. In addition, examples of the proteins include, but are not limited to, collagen and a hydrolysate thereof which is a gelatin, proteoglycan, fibronectin, vitronectin, laminin, entactin, tenascin, thrombospondin, von Willebrand factor, osteopontin, fibrinogen, and the like. Preferably, microspheres including a material that can be degraded by cells are suitable for the present invention. More preferred are microspheres including gelatin. In order to further enhance cell culture and proliferation, the microsphere surface can be coated or immobilized with a cell adhesion peptide and a cell adhesion protein such as collagen, fibronectin, vitronectin, laminin, and glycosaminoglycan.

Examples of the chemical cross-linker which can be used include condensing agents which create chemical bonds among water-soluble carbodiimide such as EDC, propylene oxide, diepoxy compounds, hydroxy groups, carboxyl groups, amino groups, thiol groups, imidazole groups, and others. Preferred is glutaraldehyde. In addition, chemical cross-linking of gelatin can be carried out by heat treatment, UV irradiation, or electron beam irradiation, etc. Also, such cross-linking treatments can be combined.

The degree of cross-linking of gelatin can be appropriately selected depending on a desired water content, that is, a hydrogel bioabsorbable level. The ranges of concentrations of the gelatin and the cross-linker at the time of the gelatin hydrogel preparation are preferably a gelatin concentration of 1 to 20 w/w% and a cross-linker concentration of 0.01 to 1 w/w%, respectively. Cross-linking reaction conditions can be carried out, but are not particularly limited to, for example, at 0 to 40°C and for 1 to 48 hours. Generally speaking, as the concentrations of gelatin and a cross-linker and their cross-linking time become larger, the degree of cross-liking of hydrogel increases and the bioabsorbable property of gelatin hydrogel decreases. Alternatively, thermal dehydration cross-linking may be carried out under reduced pressure at a high temperature. The thermal dehydration cross-linking can be performed, for example, under reduced pressure of about 0.1 Torr for 1 to 48 hours at 80 to 160°C, and preferably 120 to 140°C.

Gelatin hydrogel microspheres can be prepared by the steps of: fitting a agitator motor (e.g., THREE-ONE MOTOR, EYELA miniD.C. stirrer, manufactured by Shinto Scientific Co., Ltd.) and a Teflon (R) -made propeller to a three-neck round-bottom flask; adding a gelatin solution into a device equipped with the flask; adding thereto oil such as olive oil; stirring the mixture at about 200 to 600 rpm to yield a water in oil emulsion; and adding a cross-linker solution to the emulsion to form cross-linking between gelatin molecules. Alternatively, the gelatin solution beforehand emulsified in olive oil (e.g., by using a vortex mixer: Advantec TME-21, a homogenizer: polytron PT10-35, etc.) is dropped into olive oil, and the particularized W/O emulsion is prepared. A cross-linker solution may be added in this emulsion to carry out a cross-linking reaction. After collected by centrifugation, the resulting gelatin hydrogel microspheres are washed with acetone and ethyl acetate, etc. In addition, the gelatin hydrogel microspheres are immersed in 2-propanol and ethanol, etc., to terminate the cross-linking reaction. The resulting gelatin hydrogel microspheres are washed with, in the order of 2-propanol, distilled water containing Tween 80, and distilled water, etc. Then, the microspheres are used for cell culture. In the case of aggregation of the gelatin hydrogel microspheres, a surfactant, for example, may be added or sonication (preferably under cooling within about 1 minute) may be carried out.

The average microsphere size of the resulting gelatin hydrogel microspheres varies depending on a gelatin concentration at the time of preparation of the above-described microspheres, a volume ratio of a gelatin solution to olive oil, and a stirring rate. In general, the microsphere size is 500 nm to 1000 µm. The microspheres at an appropriately required size may be sieved out and used depending on the purpose. As used herein, the term "microsphere size" may be referred to as the term "size of microsphere". The terms "microsphere size" and "size of microsphere" are interchangeable. Further, the pre-emulsification can produce microparticulate gelatin hydrogel microspheres having a size of microsphere of 50 nm to 20 µm. Moreover, gelatin can be subjected to phase separation from an aqueous solution state, which causes the gelatin to be self-assembled to yield microspheres having a size of 50 nm to 1 µm. The phase separation can be achieved by a publicly known technique such as addition of the second component, a pH of the aqueous solution, and a change in its ion strength. As used herein, the size of microsphere is preferably about 50 nm to 1000 µm, more preferably about 500 nm to 1000 µm, still more preferably about 1 µm to 200 µm, and still more preferably about 10 µm to 160 µm.

Examples of another method for preparing gelatin hydrogel microspheres include following methods. Olive oil is added in a similar device as described in the above method, and is stirred at a rate of about 200 to 600 rpm. Next, a gelatin solution is added dropwise thereto to prepare a W/O emulsion. After the emulsion is cooled, acetone and ethyl acetate, etc., are added while stirring. Then, centrifugation is performed to collect uncrosslinked gelatin microspheres. The collected gelatin microspheres are washed further with acetone and ethyl acetate, etc., subsequently washed with 2-propanol and ethanol, etc., and dried. At this stage, microspheres having a appropriately required size may be sieved out depending on the purpose. This dried gelatin microspheres are suspended in a 0.1% Tween 80-containing cross-linker solution. The mixture is gently stirred and subjected to a cross-linking reaction. Then, depending on the cross-linker used, the mixture is washed with a 0.1 % Tween 80-containing 100 mM glycine or a 0.1 % Tween 80-containing 0.004N HC1, etc., to terminate the cross-linking reaction. Finally, gelatin hydrogel microspheres can be prepared. The gelatin hydrogel microspheres as obtained in this method have an average microsphere size similar to that of the above method.

Gelatin hydrogel microspheres of the present invention can be lyophilized, sterilized and used. The gelatin hydrogel microspheres can be lyophilized using, for example, the following steps of: adding the microspheres to distilled water; freezing the microspheres in liquid nitrogen for 30 minutes or more or at -80°C for one hour or more; and thereafter drying the microspheres with a lyophilizer for 1 to 3 days.

Cells are cultured in a regular cell culture medium with hydrogel microspheres such as gelatin hydrogel microspheres as prepared according to the above manner. Accordingly, a microsphere-containing cell aggregate of the present invention can be formed. Any cell can be used as a cell which is used in the present invention if the cell is a regular culture cell. The cell may be a cell line or a primary cultured cell. Preferable examples of the cell which can be cultured according to a method of the present invention include, in particular, stem cells such as tissue stem cells, e.g., a bone marrow-derived undifferentiated mesenchymal stem cell, a hematopoietic stem cell, a vascular stem cell, a neural stem cell, a small intestinal stem cell, an adipose stem cell, a skin stem cell, a periodontal tissue stem cell, a ciliary body stem cell, a corneal limbus stem cell, and a visceral stem cell, and pluripotent stem cells including ES cells and iPS cells. As to culture conditions such as a medium composition and a culture temperature, conditions which have been used for regular culture of these cells can be employed. When a microsphere-containing cell aggregate is cultured in accordance with the present invention, in order to prevent cells from proliferating by adhering to the culture plate surface, it is preferable to culture the cells by using a culture plate coated with polyvinyl alcohol, etc., or a substrate having characteristics of low protein adsorption or low cell attachment. The proportion of the number of microspheres to the number of cells varies depending on a microsphere diameter or cellular property. In general, the proportion of the number of microspheres to the number of cells is, but is not limited to, for example 0.1 to 30, preferably 0.3 to 10, and more preferably 0.5 and 5.

A microsphere-containing cell aggregate according to the present invention can be cultured for 7 to 40 days or more while appropriately changing a medium. As demonstrated in the following Examples, in a microsphere-containing cell aggregate of the present invention, not only cells residing on the aggregate surface but also cells residing inside can survive and proliferate. The microsphere-containing cell aggregate can be proliferated at a diameter of 50 µm to 3000 µm. Depending on the cases, the cell aggregate can be grown at a size of about 5000 µm. Although the results are different depending on a cell type and a culture method, formation of an aggregate at a diameter of 1500 µm can be achieved for 28-day culture. This seems to be because the presence of the microspheres in the cell aggregate increases substance diffusion between the inside and the outside of the aggregate, and improves nutrition and oxygen supply to the cells and waste product excretion.

The pattern of mixing the cell aggregate and the microspheres differs depending on the microsphere size. In the case of a microsphere at a size of 100 µm or more, at fast, cells proliferate on the microsphere surface. Continuous culture causes formation of a cell aggregate between the microspheres. In the case of a microsphere at a size of 20 to 100 µm, a cell aggregate is formed between the microspheres at the initial stage. Then, as a time passes, a phenomenon that the cell aggregate incorporates the microspheres is observed. If the size of microsphere is 5 to 20 µm, a microsphere-containing cell aggregate is formed from the initial stage of its culture. As illustrated in the above, any size of microsphere allows for formation of a cell aggregate. However, its process varies and the size of microsphere can be thus selected depending on the final use purpose for the cell aggregate.

In a preferable embodiment of the present invention, a cell growth factor is incorporated into hydrogel microspheres such as the gelatin hydrogel microspheres as obtained in the above-described manner. Then, cells can be cultured with these microspheres. The cell growth factor may employ any protein as long as the protein has an effect of promoting cell proliferation or differentiation. Examples of the cell growth factor can include a basic fibroblast growth factor (bFGF), an acidic fibroblast growth factor (aFGF), a platelet-derived growth factor (PDGF), transforming growth factor β1 (TGF-β1), a vessel endothelial cell growth factor (VEGF) and a connective tissue growth factor (CTGF), proteins having characteristics of inhibiting apoptosis, and peptides having the above characteristics. Hydrogel microspheres such as cell-growth-factor-containing gelatin hydrogel microspheres can be used, which promotes cell proliferation or differentiation. Other than a cell growth factor, it is possible to use a drug (e.g., a low-molecular-weight drug, a peptide drug, a nucleic acid drug) having an effect of promoting cell proliferation or differentiation, an effect of increasing a metabolic activity, or an effect of inhibiting apoptosis, the drug being incorporated into the microspheres.

Hereinafter, the present invention is specifically described by referring to Examples. However, the present invention is not limited to these Examples.

### Example 1

A fixed stirring motor (THREE-ONE MOTOR, manufactured by Shinto Scientific Co., Ltd.) was fitted with a Teflon(R)-made stirring propeller, and all were fixed to a 1000-ml round-bottom flask. Then, 375 ml of olive oil was added to the flask, and 10 ml of an alkali-treated gelatin solution (the concentration of about 10%) having an isoelectric point of 4.9 was added dropwise while stirring at 37°C and 420 rpm to prepare a W/O emulsion. After the emulsion was stirred for 10 minutes, the flask was cooled to 4°C and the emulsion was then stirred for 30 minutes. After cooling, 100 ml of acetone was added and the mixture was stirred for 1 hour. Then, centrifugation was performed to collect gelatin hydrogel microspheres. The collected hydrogel microspheres were washed with acetone and further with 2-propanol to yield uncrosslinked gelatin hydrogel microspheres. These hydrogel microspheres were dried and stored at 4°C.

Thereafter, 500 mg of the dried uncrosslinked gelatin hydrogel microspheres were suspended in 100 ml of 0.05% glutaraldehyde containing 0.1 % Tween 80, and the mixture was gently stirred at 4°C for 24 hours to carry out a gelatin cross-linking reaction. After the reaction was completed, crosslinked gelatin hydrogel microspheres were collected by centrifugation. Then, the microspheres were washed with 100 mM glycine at 37°C for 1 hour to terminate the cross-linking reaction. After the reaction was terminated, the crosslinked gelatin hydrogel microspheres were washed three times with distilled water, and were then lyophilized to yield dried crosslinked gelatin hydrogel microspheres (the average diameter of 10 µm). When these microspheres were swelled with water, the water content was about 91.5%.

Bone marrow-derived undifferentiated mesenchymal stem cells (MSC) were isolated from a rat bone marrow by using a general method, and were proliferated by culturing them. Next, the MSCs and gelatin hydrogel microspheres were mixed at various proportions, and were cultured at 37°C for 14 days. Cells were cultured using a Dulbecco's MEM medium containing 10 vol% fetal calf serum as a culture medium, and the medium was changed every three days. At that time, in order to decrease cell adhesiveness, 1% polyvinyl alcohol (manufactured by UNITIKA LTD.; the polymerization degree was 1.800; the saponification degree was 88.0%) solution was poured into a culture plate, and the solution was left at 37°C for 15 minutes. Then, the plate was washed twice with a phosphate buffer (PBS, pH 7.4) to prepare a culture plate coated with polyvinyl alcohol. Use of this culture plate causes MSCs not to adhere to the plate surface, and promotes adhesion on the mixed microspheres or between the cells.

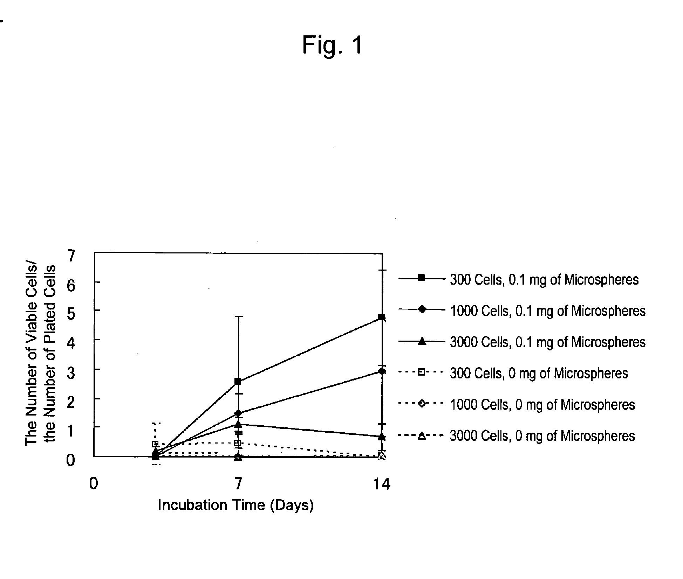

As a result, an MSC aggregate uniformly containing the microspheres was formed. As a control, in the case without having the microspheres, a microsphere-free cell aggregate was observed to be formed. The viable cell counts were quantified using a WST-8 reagent, and a ratio of the number of viable cells to the number of plated cells was calculated. A microsphere-containing MSC aggregate had a higher viable cell count than a microsphere-free cell aggregate, which indicated promotion of cell proliferation (Figure 1). In the case without having the microspheres, a cell aggregate was formed at the initial stage. As a time passed, the size of the aggregate became larger. Because of insufficient supply of nutrients and enzymes and poor excretion of waste products, the cells seemed to be die.

### Example 2

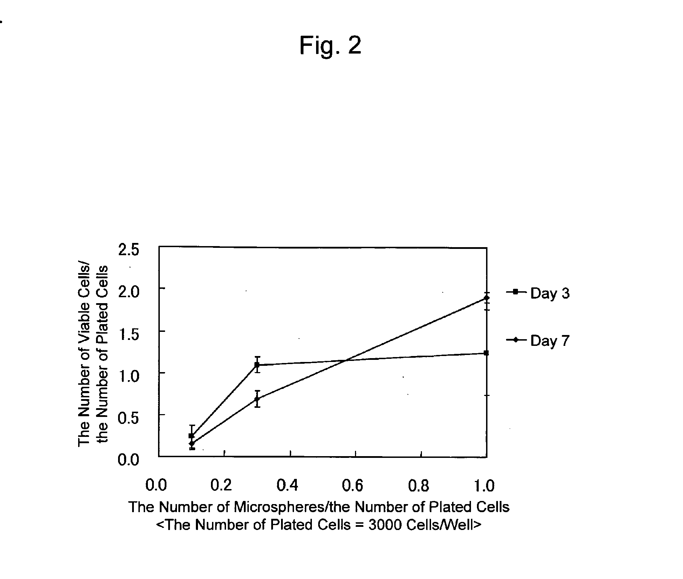

By changing a ratio of the number of microspheres to the number of MSCs, a cell aggregate was produced in a similar manner. As a result, when a ratio of the number of microspheres/the number of cells was 0.3 or more, the viable cell count increased with increasing culture period (Figure 2). That is, as a ratio of microspheres/cells became larger, the cell viability increased. This result seems to be due to the presence of the microspheres within the cell aggregate. Since substance diffusion between the inside and the outside of the aggregate increases, nutrition and oxygen supply to the cells and waste product excretion improve.

### Example 3

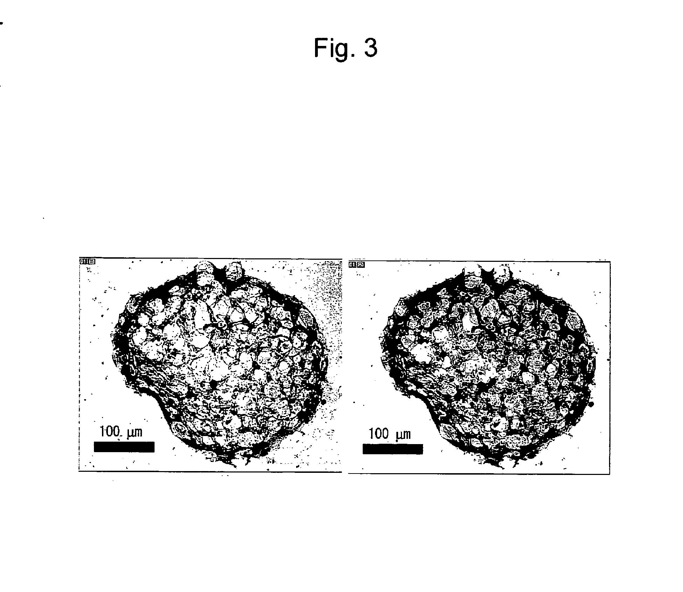

In a manner similar to that of Example 2, a microsphere-containing MSC aggregate was produced at a ratio of the number of microspheres/the number of cells of 0.5. BrdU incorporation into this aggregate was investigated. At day 6 of cell culture, BrdU was added to a medium. At day 7, a cell aggregate was collected, and frozen sections were prepared. A section having a maximum area through an aggregate center portion was subjected to immunostaining using an anti-BrdU antibody. The stained portion represented a site where BrdU was highly incorporated into a nucleus. This indicated proliferation of cells. The results demonstrated that BrdU was incorporated into cells on the surface and inside of the aggregate. Accordingly, the cells residing inside the aggregate were found to proliferate (Figure 3). On the other hand, a microsphere-free MSC aggregate was cultured under the identical conditions. Then, the cell aggregate was formed, and BrdU incorporation was investigated. The results demonstrated that cells in a vicinity of the aggregate surface incorporated BrdU, but no incorporation was found in cells residing inside the aggregate. The cells residing inside the aggregate were found dead.

### Example 4

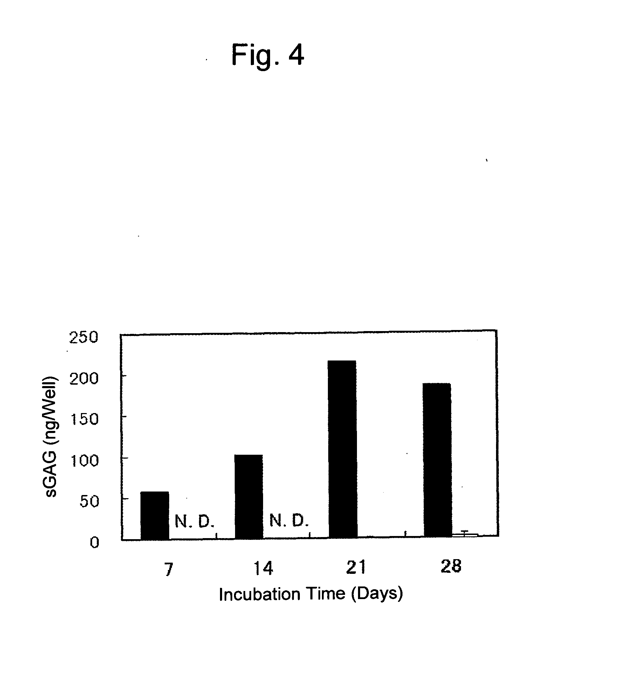

Under the same conditions as in Example 3, a microsphere-containing MSC aggregate was formed. Next, this MSC aggregate was cultured in a chondrocyte differentiation medium (1% FCS-containing high glucose DMEM medium containing 1 wt% transferrin and insulin, 1 mM pyruvic acid, 100 mM ascorbic acid-2-phosphate, 100 nM dexamethasone, 10 ng/ml transforming growth factor (TGF) β1) to induce chondrocyte differentiation. As a control, a microsphere-free MSC aggregate was used. The chondrocyte differentiation was evaluated using a sulfated glycosaminoglycan (sGAG) production. sGAG was measured as follows. Cells were lysed in a papain solution (i.e., a phosphate buffer (pH 6.5) containing 2 mM dithiothreitol and 1 mM EDTA) at a concentration of 300 µg/ml. Then, 1,9-dimethylmethylene blue (225 µL) was added to this cell lysate (25 µL), and absorbance at 525 nm was measured. A standard curve was drawn using chondroitin sulfate, and sGAG was quantified by using the standard curve. The results demonstrated that in a microsphere-containing MSC aggregate, sGAG secretion was detested over 28 days. In contrast, in a microsphere-free aggregate, no sGAG secretion was observed. These results indicated that in the microsphere-containing cell aggregate, chondrocyte differentiation was efficiently induced (Figure 4). This seems to be because in the inside of the aggregate, substance diffusion causes a good cell viability status to be maintained.

### Example 5

A TGF-β1 solution was added dropwise to the dried product of the gelatin hydrogel microspheres as produced in Example 1, and the mixture was left at 25°C for 1 hour to make TGF-β1 impregnated in the gelatin hydrogel microspheres. An amount of TGF-β1 impregnated in the gelatin hydrogel microspheres which are mixed with cells and cultured is 1 ng. By using these microspheres, an MSC aggregate was formed. In a manner similar to that of Example 4, chondrocyte differentiation was induced and the chondrocyte differentiation was examined by measuring sGAG secretion. The results demonstrated that in the case of having microspheres impregnated with TGF-β1, the chondrocyte differentiation was observed and the sGAG production significantly increased. This production was shown to exhibit a higher value than that of the microsphere-containing cell aggregate in Example 4. As a control, the same amount (1 ng) of TGF-β1 was added to a medium for a microsphere-free cell aggregate, and the experiments were carried out. In this case, however, no chondrocyte differentiation was observed.

A MSC aggregate containing the microspheres impregnated with TGF -β1 was cultured for chondrocyte differentiation, and the cell aggregate was then stained with alcian blue. The results verified the presence of a cartilage matrix which had been uniformly stained inside the aggregate with alcian blue. In contrast, a microsphere-free aggregate was found dead. These results indicated that: the presence of the microspheres enhances diffusibility of substances between the inside and the outside of the cell aggregate, thereby improving cellular conditions; and local supply of TGF-β1 from the microspheres further promotes the chondrocyte differentiation.

### Example 6

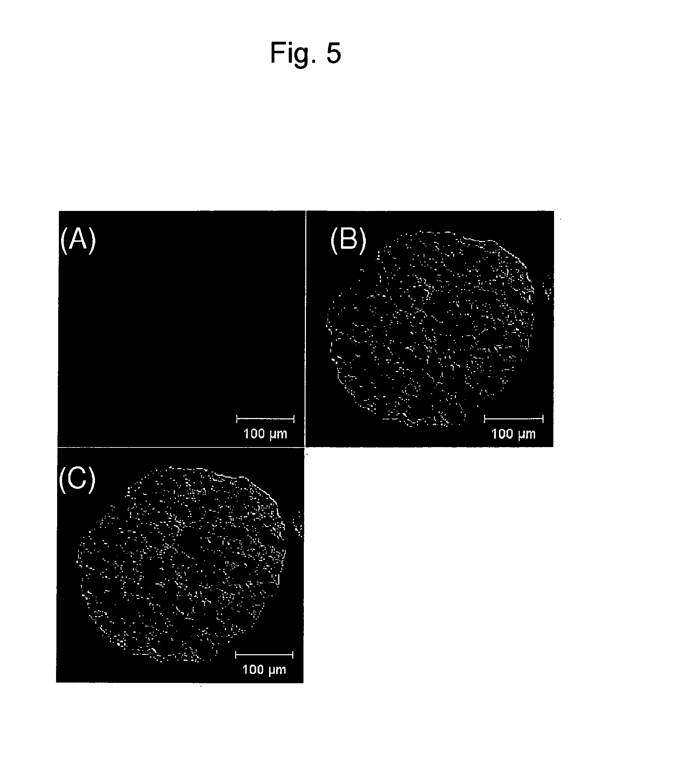

In a manner similar to that of Example 1, gelatin hydrogel microspheres were prepared. However, at the time of the microsphere preparation, thermal dehydration cross-linking (under conditions at 140°C, for 48 hours, at 0.1 Torr) was employed. The size of microsphere at the time of water swelling was 17.6 µm ± 7.4 µm (the size of microsphere in the dried state was 10 µm). The number of MSCs was set to 1 x 10⁴ cells/well, and the number of microspheres was set to 1 x 10⁴ microspheres/well. These MSCs were cultured in a manner similar to that of Example 1, and observed at day 7 of culture. Frozen sections of the microsphere-containing cell aggregate were prepared, and cell nuclei were stained (with TO-PRO-3 fluorescent dye for staining cell nuclei, manufactured by Invitrogen, Inc.). After that, the sections were observed with a confocal fluorescence microscope and a light microscope. Figure 5 is nuclear staining images of cross sections of the cell aggregate: (A) a confocal fluorescence microscope image (red staining; cell nuclei), (B) a light microscope image, (C) a superimposed image. The results revealed that even in the inside of the aggregate, viable cells were present, and the cells and the microspheres were uniformly mixed.

### Example 7

The same method as in Example 1 except the cross-linking method was applied to a preparation of gelatin hydrogel microspheres, but the stirring speed at the time of the microsphere preparation was changed to 200, 300, and 450 rpm. After washed with acetone and 2-propanol and dried, the respective microspheres were sieved out to yield three kinds of uncrosslinked microspheres having an average microsphere size of 10 µm, 26 µm, and 44 µm in the dried state. These uncrosslinked microspheres were subjected to thermal dehydration cross-linking at 140°C for 48 hours under reduced pressure of 0.1 Torr to crosslink gelatin, and gelatin hydrogel microspheres were then prepared. The resulting microspheres were swelled with distilled water. At this time, the microsphere sizes were 17.6 ± 7.4 µm, 47.9 ± 22.2 µm, and 106.8 ± 17.8 µm, and the water content was 92.0%.

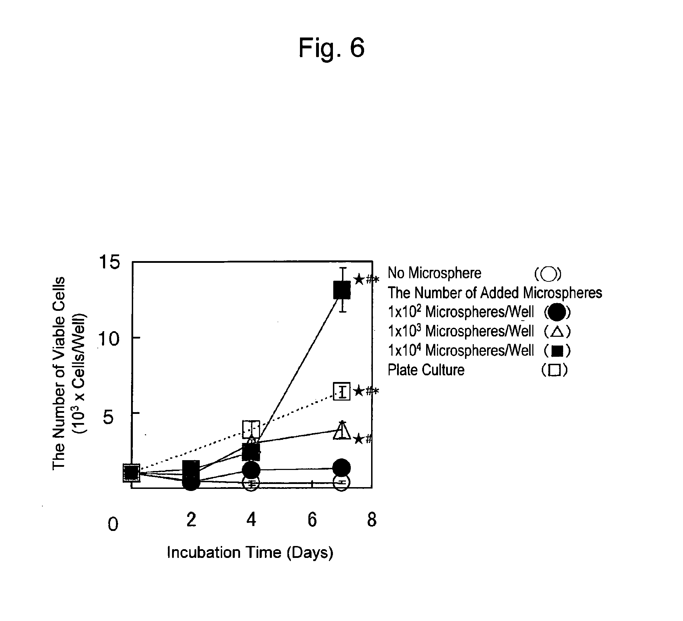

These three kinds of gelatin hydrogel microspheres at different sizes were used to culture with MSCs in the same manner as in Example 1. Then, a microsphere-containing MSC aggregate was prepared. Figure 6 shows viable cell counts when the microspheres at a size of 106 nm were used for culture; the number of plated cells was set to 1 x 10³ cells/well; the number of added microspheres was set to 1 x 10² microspheres/well, 1 x 10³ microspheres/well, or 1 x 10⁴ microspheres/well. As a control, a microsphere-free aggregate (no microsphere) and a two-dimensional plate culture (plate culture) utilizing a regular culture substrate were used. The results revealed that in the microsphere-free cell aggregate, cells failed to proliferate and was going to die. In contrast, in the microsphere-containing cell aggregate, cells were found to proliferate. By selecting an MSCs/microspheres ratio, the cell aggregate was found to proliferate faster than that of the plate culture.

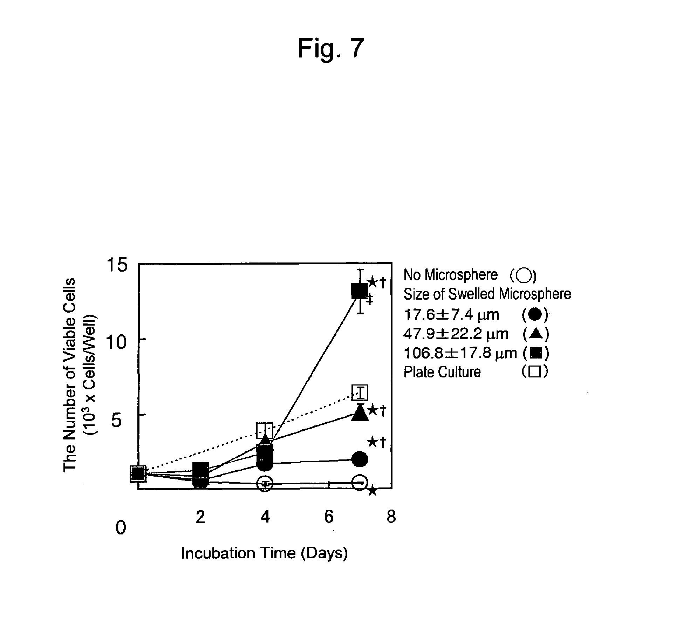

Figure 7 shows viable cell counts when the size of microsphere varied for culture; the number of plated cells was set to 1 x 10³ cells/well; and the number of added microspheres was set to 1 x 10⁴ microspheres/well. As a control, a microsphere-free aggregate (no microsphere) and a two-dimensional plate culture (plate culture) utilizing a regular culture substrate were used. The results demonstrated that regardless of the size of microsphere, in any case, the cellular proliferation significantly increased compared to that of the microsphere-free cell aggregate. In addition, when the size of microsphere was 106 µm (the size was 44 µm in the dried state), the cells were found to proliferate significantly faster than those of the plate culture.

### Example 8

Culture experiments were carried out in a manner similar to that of Example 7. Glucose consumption was selected as an index for cellular conditions associated with culture. By using Glutest Neo Super (manufactured by ARRAY, Inc.), the cellular glucose consumption was quantified. The number of cells was 1 x 10⁴ cells/well, and the number of microspheres was 1 x 10⁴ microspheres/well. The size of microsphere used was 17.6, 47.9, and 106.8 µm (the size of microsphere in the dried state was 10, 26, and 44 µm).

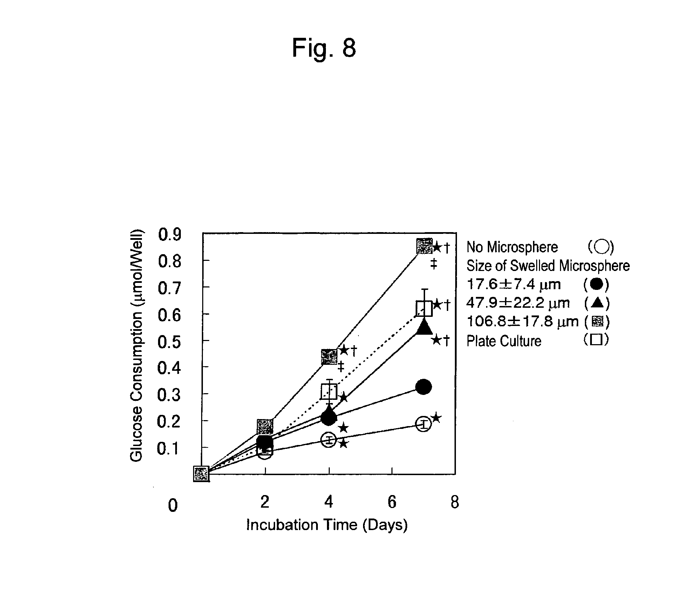

Figure 8 shows the results. In any case, as the culture proceeded, more glucose was consumed. This indicated cell growth. Regardless of the size of microsphere used, the microsphere-containing cell aggregate exhibited a significantly higher value for glucose consumption than the microsphere-free cell aggregate. In addition, when the size of microsphere was 106 µm, the highest consumption was exhibited. This indicated that the consumption was significantly high and the cellular growth conditions were good, compared with those of the plate culture.

### Example 9

Culture experiments were carried out in a manner similar to that of Example 7. As an index for cellular energy metabolism, a ratio of L-lactic acid production/glucose consumption was calculated. The lower this index is, the more rapidly the aerobic energy metabolism proceeds in cells. This means a better oxygen condition in the cells. Production of L-lactic acid was quantified using an E-kit (manufactured by R-Biopharm AG). The number of cells was 1 x 10³ cells/well, and the number of microspheres was 1 x 10⁴ microspheres/well. The size of microsphere used was 17.6, 47.9, and 106.8 µm (the size in the dried state was 10, 26, and 44 µm).

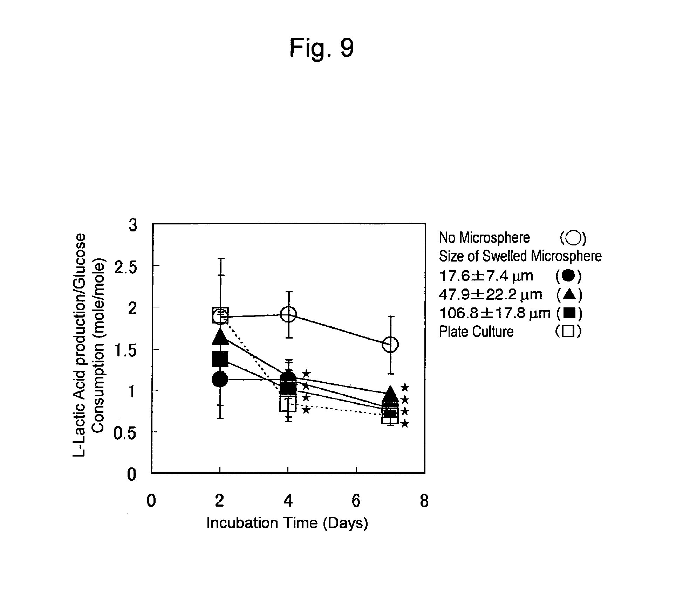

Figure 9 shows the results. Regardless of the size of microsphere used, the microsphere-containing cell aggregate exhibited a significantly lower ratio of L-lactic acid production/glucose consumption than the microsphere-free cell aggregate. Further, this value was a level equivalent to that of the plate culture. This indicates that inclusion of the microspheres causes better oxygen conditions inside the cell aggregate, which results in increased cellular aerobic energy metabolism. This value was a level equivalent to that of the plate culture which was considered superior to three dimensional cell aggregate culture under conditions having oxygen and nutrients. The diameter of the cell aggregate including microspheres at a size of 106 µm was 500 µm (at day 4 of culture) and 600 µm (at day 7 of culture). Usually, the distance at which oxygen is supplied by diffusion is 100 µm. Consequently, when the size of the cell aggregate becomes larger than 100 µm, oxygen-depleted state prevails inside the cell aggregate. This is known to cause cells to be die. However, formation of the cell aggregate by inclusion of the microspheres can create an environment which can efficiently supply nutrients and oxygen to the inside of the aggregate. Even inside of the cell aggregate having a large size of 500 and 600 µm, the cells are not die, and aerobic metabolism can proceed.

All the publications, patents, and patent applications cited in this specification are herein incorporated by reference in its entirety.

## Claims

1. A microsphere-containing cell aggregate comprising: hydrogel microspheres obtained by chemical cross-linking of one or more water-soluble synthetic macromolecules selected from the group consisting of a water-soluble synthetic polymer, a polysaccharide, and a protein; and cells.

2. The microsphere-containing cell aggregate according to Claim 1, wherein the water-soluble synthetic polymer is selected from the group consisting of polyacrylamide, polyacrylic acid, polyhydroxyethyl methacrylate, and polyvinyl alcohol.

3. The microsphere-containing cell aggregate according to Claim 1 or 2, wherein the polysaccharide is selected from the group consisting of glycosaminoglycan, starch, glycogen, agarose, pectin, and cellulose.

4. The microsphere-containing cell aggregate according to any one of Claims 1 to 3, wherein the protein is selected from the group consisting of collagen and a hydrolysate thereof which is a gelatin, proteoglycan, fibronectin, vitronectin, laminin, entactin, tenascin, thrombospondin, von Willebrand factor, osteopontin, and fibrinogen.

5. The microsphere-containing cell aggregate according to any one of Claims 1 to 4, comprising: gelatin hydrogel microspheres obtained by inter-molecular chemical cross-linking of gelatin; and cells.

6. The microsphere-containing cell aggregate according to any one of Claims 1 to 5, wherein a cell adhesion protein or a cell adhesion peptide is coated or immobilized on the surface of the hydrogel microspheres.

7. The microsphere-containing cell aggregate according to Claim 6, wherein the cell adhesion protein or the cell adhesion peptide is selected from the group consisting of collagen, fibronectin, vitronectin, laminin, and glycosaminoglycan.

8. The microsphere-containing cell aggregate according to any one of Claims 1 to 7, wherein the hydrogel microsphere has a microsphere size of 50 nm to 1000 µm.

9. The microsphere-containing cell aggregate according to Claim 8, wherein the hydrogel microsphere has a microsphere size of 100 µm or more, between 20 and 100 µm, or between 5 and 20 µm.

10. The microsphere-containing cell aggregate according to any one of Claims 1 to 9, wherein the number of the hydrogel microspheres per cell is between 0.1 and 30.

11. The microsphere-containing cell aggregate according to any one of Claims 1 to 9, wherein the number of the cells per 0.1 mg of the hydrogel microsphere is between 300 and 3000.

12. The microsphere-containing cell aggregate according to any one of Claims 1 to 11, wherein the hydrogel microsphere comprises a cell growth factor.

13. The microsphere-containing cell aggregate according to any one of Claims 1 to 12, wherein when the hydrogel microsphere comprises gelatin, a range of concentration of gelatin is 1 to 20 w/w% and a range of concentration of a cross-linker is 0.01 to 1 w/w%.

14. The microsphere-containing cell aggregate according to any one of Claims 1 to 13, wherein the cell is a stem cell.

15. A method for producing the microsphere-containing cell aggregate according to any one of Claims 1 to 14, the method comprising the step of culturing cells in a culture medium comprising hydrogel microspheres.
